# EUROPEAN PATENT APPLICATION

(11) **EP 4 137 580 A1**
(43) Date of publication of application: **22.02.2023**
(21) Application number: 21192309.9
(22) Date of filing: 20.08.2021
(51) Int. Cl.: C12Q 1/6806, C12Q 1/6883

(54) **SCREENING FOR KARYOTYPES WITH ABBERATIONS IN THE GONOSOMES BY DIRECT QUANTITATIVE FLUORESCENT PCR**

(71) Applicant: Eluthia GmbH, 35394 Gießen (DE)
(72) Inventor: Schäfer, Ramón Enríquez, 35394 Gießen (DE); Becker-Follmann, Johannes, 66133 Saarbrücken (DE)
(74) Representative: Ullrich & Naumann PartG mbB

(57) **Abstract**

The present invention relates to a method for the *in vitro* detection of karyotypes with aberrations of the gonosomes or mosaics of said karyotypes in a human by quantitative fluorescence polymerase chain reaction (QF-PCR) using short tandem repeat (STR) markers and X-linked segmental duplication (SD) markers comprising the following steps: a. providing a sample previously obtained from the human; b. adding the sample to a PCR buffer system to and performing multiplex QF-PCR directly with the sample in a PCR buffer system using primer pairs for at least two X-linked SD markers and primer pairs for at least two STR markers located on the X-chromosome and to obtain amplification products; c. separating the marker amplification products; d. determining the peak heights or peak area of the separated amplification products; e. calculating the peak height ratio or peak area ratio for each X-linked SD marker and comparing calculated ratios with a predefined range obtained from samples of karyotype 46,XX or 46,XY; wherein a peak height ratio or peak area ratio of the X-linked SD marker outside of the predefined range obtained from samples of karyotype 46,XX is indicative of a karyotype with aberrations of the X chromosome or a mosaic of said karyotype.

## Description

### FIELD OF THE INVENTION

The present invention relates to a method for the *in vitro* detection of karyotypes with aberrations of the gonosomes or mosaics of said karyotypes in a human by quantitative fluorescence polymerase chain reaction (QF-PCR) using short tandem repeat (STR) markers and X-linked segmental duplication (SD) markers.

### BACKGROUND OF THE INVENTION

The aim of newborn screening is to detect treatable disease states at an early stage in order to be able to initiate therapy in good time. Currently, 16 so-called target diseases are included in the extended newborn screening (ENS) as defined in the Children's Guideline of the Federal Joint Committee on the Early Detection of Diseases in Children (Children's Guideline).

The majority of detections are mass spectrometric methods, followed by immunological and biochemical methods. Only the detection of two diseases, namely Severe Combined Immunodeficiencies (SCID) (see Borte et al. 2014) and spinal muscular atrophy (SMA) (see Czibere et al 2020) are performed by DNA analysis.

For inclusion in the extended newborn screening, not only medical criteria apply, such as the offer of an effective therapy, but also economic considerations. DNA analytical procedures generally require more effort and costs. For comparison, the costs of a mass spectrometric analysis are about 2 to 3 Euro (2021), while even a cheap DNA preparation from blood substances is hardly feasible for 2 Euro per test person.

The frequency of all disorders screened in extended newborn screening is approximately: 1/1000 newborns (AWMF Leitlinien 2020). Gonosomal aneuploidies occur at a frequency of approximately 1 in 500 newborns, including the 45,X0 karyotype with approximately 1 in 2500 newborns (Hassold, et al. 2001). The Absence of the second X chromosome in whole or in part leads to the medical condition of Turner syndrome. The majority of cases, approximately 40%, are characterized as 45,X0. Another ca. 40 % are mosaics characterized as 45,X0/46,XX, 45,X0/46,XY and other mosaics with 46,XX/46,Xder(X) summarized as Non 46,XX (see Gravholt et al. 2017). In all cases, initiation of early therapy is necessary for normal development.

A second group is aneuploidies of the X chromosome with complete Y chromosome i.e. 47,XXY or 48, XXXY and mosaics of both, grouped together as Non 46,XY. This group is referred to as Klinefelter syndrome.

Whether a sample contains two X chromosomes (karyotype 46,XX) or one X chromosome and one Y chromosome (karyotype 46,XY) can be quantified absolutely by a karyogram, the representation and counting of the chromosomes during cell division (mitosis). However, cytogenetic imaging of chromosomes is too labor intensive and costly for screening.

Alternatively, relative quantification can be performed using molecular biological methods, for example by comparing PCR amplification of a diagnostic marker, e.g. X marker, with a reference marker, e.g., an autosomal marker on, e.g., chromosome 3. Such analyses are referred to as quantitative fluorescence PCR or QF-PCR because peak heights or peak areas of the amplification products are measured after capillary gel electrophoresis and related to each other (see Mann and Ogilvie 2012).

Other methods use so-called polymorphic markers, for example short tandem repeat (STR) markers, which are short DNA sequence blocks arranged in succession that can be present in varying numbers on the chromosome. Determining the length of the PCR fragments e.g. by capillary electrophoresis, the number of block repeats can be concluded. Due to the difference in a population (polymorphism), different fragment lengths can be addressed as alleles of one locus on one chromosome, in the heterozygous state = informative state, both chromosomes can be identified as distinct. Heterozygosity (different alleles) at one STR locus therefore indicates two X chromosomes, homozygosity may mean one X chromosome. Whether it is a true homozygosity or a hemizygosity, i.e. absence of one of the X chromosomes cannot be decided at only one locus. However, if all markers are homozygous, the probability of random homozygosity can be calculated from the allele frequencies in a population to determine whether it is hemizygosity (45,X0) or random homozygosity at all loci.

A further example is the method according US2010/0062430. Said method uses QF-PCR with specific segmental duplication (SD) markers for diagnosing Turner Syndrom. SDs are two similar sequences with different fragment lengths, located on two different chromosomes. These sequences maintain the original ratio between the two different chromosomes when co-amplified using a single pair of fluorescent primers. The PCR products of different sizes are subsequently analyzed through capillary electrophoresis, and the aneuploidies are determined based on the relative dosage between the two chromosomes in a single reaction.

All of these DNA analytical solutions for genetic screening methods are based on a three-step procedure: DNA preparation from dried blood spots (DBS), amplification of the target DNA and analysis of the amplification product usually by real-time PCR. This is for example described in CN201810857790.

### SUMMARY OF THE INVENTION

The present inventors have identified an improved method for the detection of Non 46,XX or Non 46,XY karyotypes, i.e. karyotypes with aberrations of the gonosomes in a human based on QF-PCR with SD and STR markers. This method not only allows an amplification of the DNA directly from the sample without the necessity of DNA preparation decreasing the time to obtain a result and saving resources, but also highly reliably detects Non 46,XX or Non 46,XY karyotypes and even allows the determination of the karyotype or mosaic of karyotypes.

Thus, the present invention provides a method for the *in vitro* detection of karyotypes with aberrations of the gonosomes or mosaics of said karyotypes in a human by (multiplex) quantitative fluorescence polymerase chain reaction (QF-PCR) using short tandem repeat (STR) markers and X-linked segmental duplication (SD) markers comprising the following steps:
a. providing a sample previously obtained from the human;
b. adding a PCR buffer to the sample and performing multiplex QF-PCR directly with the buffered sample using primer pairs for at least two X-linked SD markers and primer pairs for at least two STR markers located on the X-chromosome and to obtain amplification products;
c. separating the marker amplification products;
d. determining the peak heights or peak area of the separated amplification products;
e. calculating the peak height ratio or peak area ratio for each X-linked SD marker and comparing calculated ratios with a predefined range obtained from samples of karyotype 46,XX or 46,XY;
wherein a peak height ratio or peak area ratio of the X-linked SD marker outside of the predefined range obtained from samples of karyotype 46,XX is indicative of a karyotype with aberrations of the X chromosome or a mosaic of said karyotype.

Due to its low cost, high-throughput eligibility and being excellent in specificity and sensitivity, not to cause further costly analysis by false positives or fail to serve their purpose by false negatives, the present method is particularly well-suited for newborn screenings.

### FIGURES

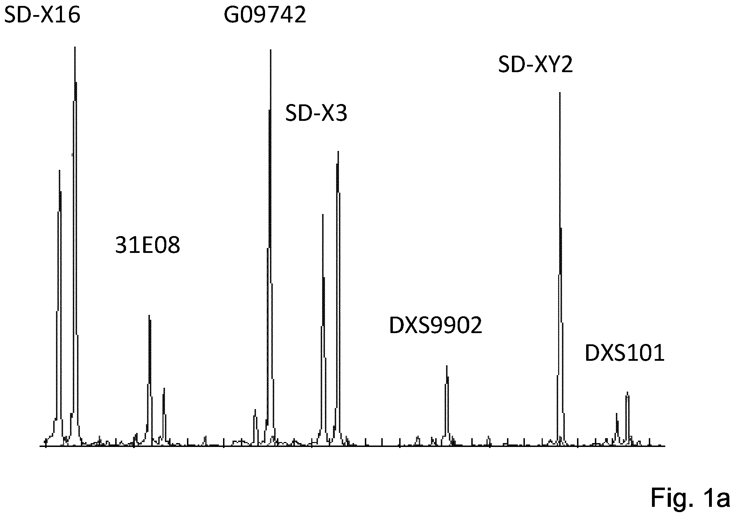

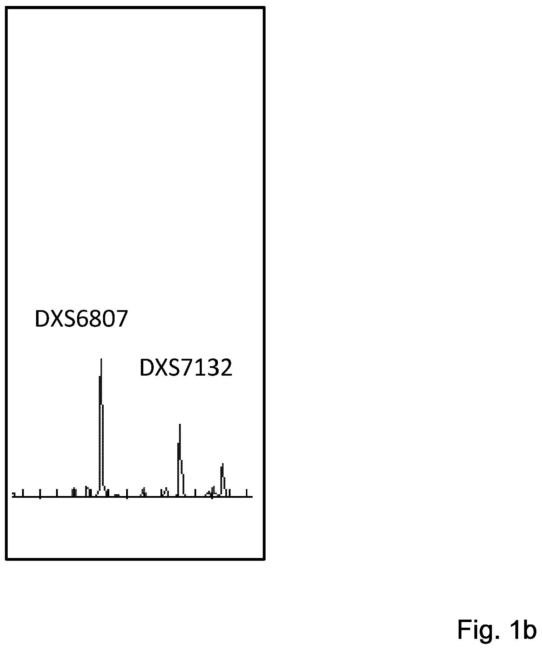

- **Fig 1**: shows the peaks of amplified DNA fragments (DNA fingerprint) of a subject with mosaic and aberrant chromosome X (mos 46,X,i(X)(q10),[28]/45,X [3] represented in two parts. Fig 1 A: fragments of lengths: 80 nt to 229 and Fig. 1 b): fragments of lengths 230 nt to 304 nt.

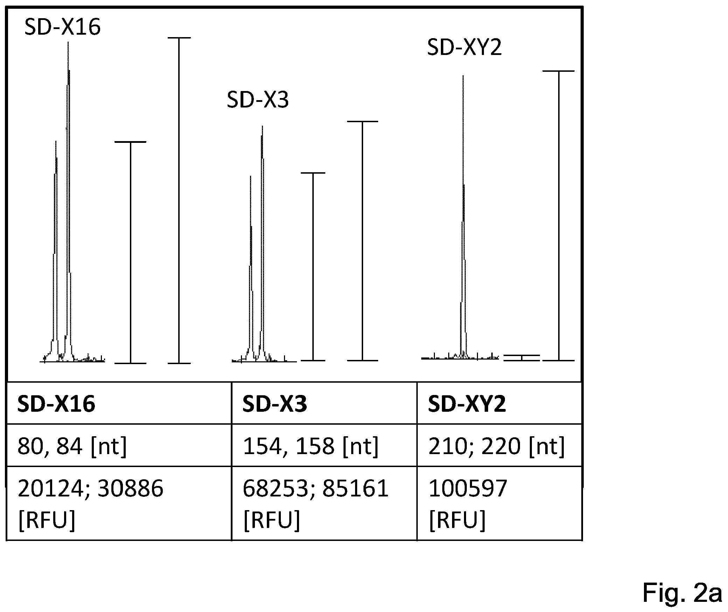

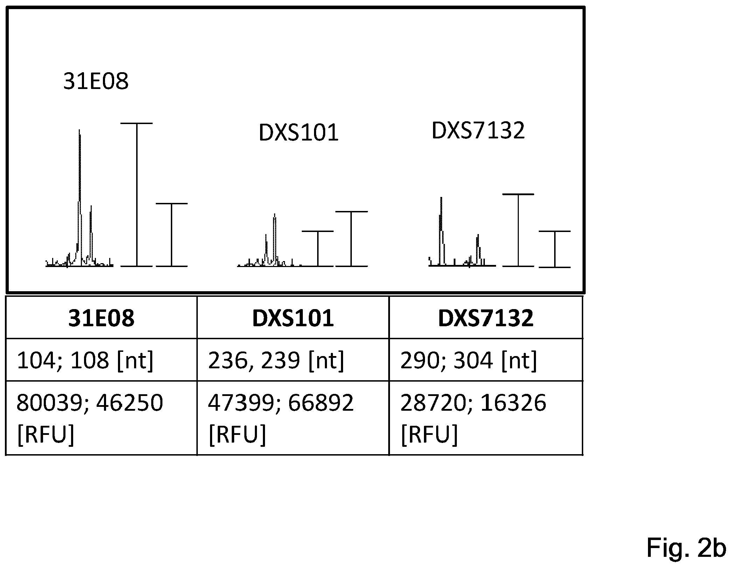

- **Fig. 2**: a) shows the data of the SD markers of a subject with allele size [nt] and the peak height as relative fluorescence units [RFU]. The bounding lines next to the marker peaks illustrate the peak heights. The calculated SD ratios from the peak heights are: X16 = 0.65, X3 = 0.80, the SD-XY value = 0, so we are dealing with suspected Non 46,XX and Non 46,XY. The predominance of the SD-X3 marker on the long arm of chromosome X indicates mosaicism with duplicated long arm. b) shows the QFtest result from one subject using three STR markers localized on the long arm of the X chromosome. All markers are heterozygous with different peak heights, indicating that the long arm of the X chromosome is present in two copies without evidence of a second copy of the short arm.

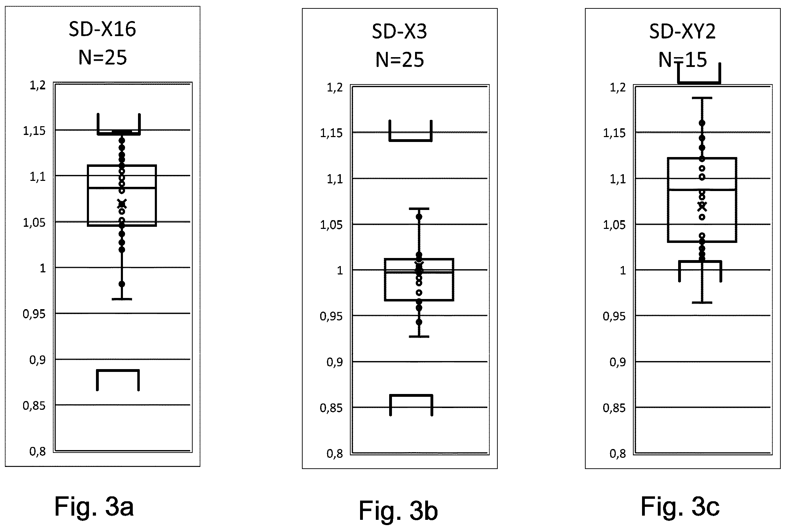

- Fig. 3: shows the peak height ratios as a box plot of diagnostic marker X against reference marker on the autosomal chromosomes of 25 healthy women with karyotype 46.XX: the median corresponds to the horizontal line in the box, the cross to the mean value, upper boundary of the box indicates the 25% quartiles, the lower boundary indicates the lower 25% quartiles, the antennas mark maximum and minimum of the data points of the test persons. The square brackets mark the outlier limit calculated according to Grubb with significance level p=0.99. All samples with data points outside the outlier limit would be considered as Non 46,XX. Fig. 3a shows the box plot of the SD-X16 markers. Mean = 1.069706747, median = 1.086801692, standard deviation: 0.071791586. Fig. 3b shows the box graph of SD-X3 markers. Mean = 1.0037197, median = 0.99694341, standard deviation: 0.04649662. Fig. 3c shows the box graph of SD-X16/SD-X3 marker ratio. Mean = 1.068853227, median = 1.087412459, standard deviation: 0.094470758.

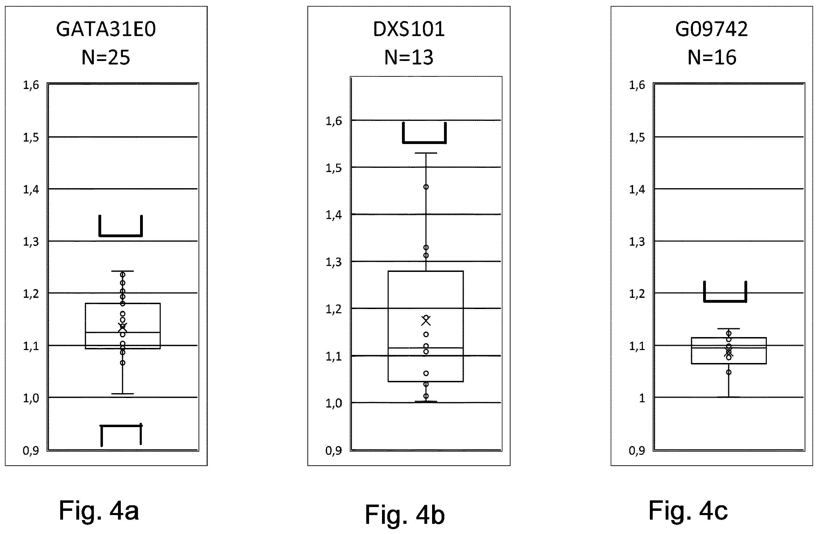

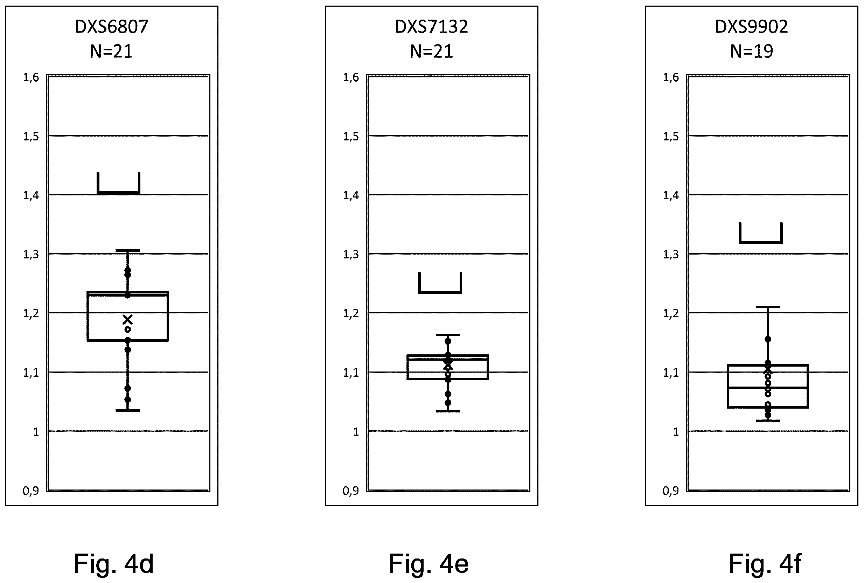

- Fig. 4: shows the peak height ratios of the alleles of the heterozygous X-linked nuclei of 25 healthy women with karyotype 46.XX plotted in a box diagram. The box heading indicates the STR marker, N indicates the number of subjects who were heterozygous for the corresponding marker. The median corresponds to the horizontal line in the box, the cross corresponds to the mean, upper boundary of the box denotes the 25% quartiles, the lower boundary denotes the lower 25% quartiles, the antennas mark the minimum and minimum of the data points. The square brackets mark the outlier limit calculated by Grubb with significance level p=0.99. All samples with data points outside the outlier limit would be considered as Non 46,XX presumably Mosa-ike 45,X0/46,XX.

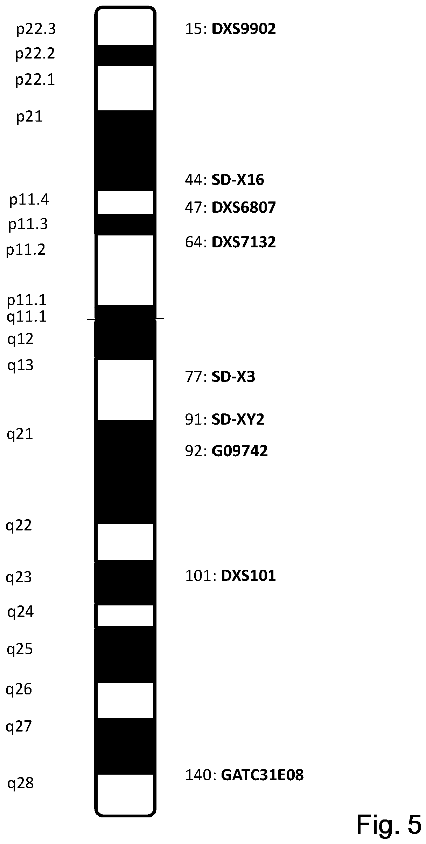

- **Fig. 5**: shows the representation of the X-chromosome at the 550 band stage. The bands are labeled p for the short arm and q for the long arm. On the right side is the position of the markers with the positions in megabase pairs from 15 (DXS9902) to 140 (GATC31E08).

### DETAILED DESCRIPTION OF THE INVENTION

In the context of the invention the following definitions are used.

The term "PCR" stands for Polymerase Chain Reaction and is a method to generate specific DNA fragments by specific binding and extension of starter molecules (primers) to a template molecule with an exponential propagation of the PCR fragment to obtain sufficient material for further analysis.

The term "marker" refers to an analyzable DNA segment at a specific locus on the chromosomes (site on the chromosomes), which can perform a labeling function for an analysis of the chromosomes.

The term "allele" as used herein refers to a sequence variant of a gene or a marker with which both X chromosomes can be distinguished. In the case of SD markers, both loci of a marker can also be considered alleles.

The term "SD marker" as used herein mean segmental duplication marker and refers to a duplicated DNA fragment mapped to different loci on different or the same chromosome. Due to sequence identity of more than 90%, an SD marker can be amplified with the same primer pair with similar efficiency by PCR. An "X-linked SD marker" as used herein is an SD marker with one copy of the DNA fragment on the X chromosome.

As used herein the term "short tandem repeat" (STR) mean stands for short consecutive sequence blocks that repeat and can be variable in number (polymorphic) in a population.

The terms "SD ratio" or "allele ratio" stand for the ratio of peak heights or peak areas of the amplified marker alleles of the higher peak related to the lower peak after measurement in a capillary gel electrophoresis.

"Dried blood spot" (DBS) testing is a form of biosampling where blood samples are blotted and dried on filter paper. Dried blood spot specimens are collected by applying a few drops of blood, drawn by lancet from the finger, heel or toe, onto specially manufactured absorbent filter paper. The blood is allowed to thoroughly saturate the paper and is air dried for several hours. Once in the laboratory, technicians separate a small disc of saturated paper from the sheet using an automated or manual hole punch, dropping the disc into a flat bottomed microtitre plate.

Quantitative Fluorescent-Polymerase Chain Reaction (QF-PCR). QF-PCR is a method able to identify and simultaneously quantify chromosome-specific DNA sequences QF-PCR relies on comparable PCR amplification efficiency of all oligonucleotides included in the reaction. PCR, an abbreviation for Polymerase Chain Reaction, is a technique to exponentially amplify a small quantity of a specific nucleotide sequence in the presence of template sequence, two oligonucleotide primers that hybridize to opposite strands and flank the region of interest in the target DNA, and a thermostable DNA polymerase. The reaction is subjected to different temperatures in cycles involving template denaturation, primer annealing, and the extension of the annealed primers by DNA polymerase until enough copies are made for further analysis. The performing of PCR analyses per se is considered well known to a skilled person, having access to reagents, apparatuses and protocols from many different suppliers.

The present invention relates to a method for the *in vitro* detection of karyotypes with aberrations of the gonosomes or mosaics of said karyotypes in a human by quantitative fluorescence polymerase chain reaction (QF-PCR) using short tandem repeat (STR) markers and X-linked segmental duplication (SD) markers comprising the following steps:
a. providing a sample previously obtained from the human;
b. adding the sample to a PCR buffer system to and performing multiplex QF-PCR directly with the sample in a PCR buffer system using primer pairs for at least two X-linked SD markers and primer pairs for at least two STR markers located on the X-chromosome and to obtain amplification products;
c. separating the marker amplification products;
d. determining the peak heights or peak area of the separated amplification products;
e. calculating the peak height ratio or peak area ratio for each X-linked SD marker and comparing calculated ratios with a predefined range obtained from samples of karyotype 46,XX or 46,XY;
wherein a peak height ratio or peak area ratio of the X-linked SD marker outside of the predefined range obtained from samples of karyotype 46,XX is indicative of a karyotype with aberrations of the X chromosome or a mosaic of said karyotype.

As shown in the examples, with the method according to the invention using QF-PCR with primers for the amplification of at least two X-linked SD markers and at least two STR markers directly for

The sample can be in principle any type of human sample. However, for screening purposes, samples easy to obtain are preferred. According to one embodiment, the sample previously obtained from the human is selected from blood, saliva and urine. In case of blood preferably dry blood is used as it easy to handle. For a screening, the preferred form of the dry blood punches from dry blood cards. In case of saliva, preferably oral mucosa swabs are used.

One important element of the method according to the invention is the right selection of the buffer. In particular, according to the obtained results the Bovine serum albumin (BSA) appears to be important. According to one embodiment, the PCR buffer system contains BSA. The BSA concentration may be in the range of concentration of 300 ng/ul to 600 ng/ul. For example, the BSA concentration may be 300 ng/ul, 350 ng/ul, 400 ng/ul, 450 ng/ul, 500 ng/ul, 550 ng/ul, or 600 ng/ul. According to a preferred embodiment, the BSA concentration is in the range of 400 ng/ul to 500 ng/ul.

The PCR buffer may be provided in the form of a PCR master mix, including the buffer the nucleotides as well as the polymerase. Suitable commercially available master mixes are for example HOT FIREPol^{®} MultiPlex Mix with 10 mM MgCl₂ (Solis Biodyne Estonia), S7Fusion (Biozym), Blutdirekt 2x PCR Mastermix (Bio&SELL), 5x Multiplex QPCR Mix (Bio&Sell), 5x Hemo KlenTaq Reaction buffer (NEB), Multiplex-PCR-Kit (Qiagen), Phusion High-Fidelity PCR Master Mix with HF Buffer (Thermofisher). Based on the experimental data, HOT FIREPol^{®} MultiPlex Mix with 10 mM MgCl2 is preferred.

Additionally, in order to obtain a good yield for both the very short and longer amplification products, the PCR parameters should be optimized. In particular, the PCR protocol contains a hot start step, a number of cycles of denaturing, annealing and extension and a final extension step.

The number of cycles is preferably in the range of 25 to 55. The number of cycles may be, for example, 25, 30, 35, 40, 45, 50, or 55. Preferably, the number of cycles is in the range of 30 to 40. More preferably, the number of cycles is in the range of 33 to 37. Most preferably in the number of cycles is 35.

According to one embodiment, the time of the final extension step is in the range of 40 to 80 min. The time of the final extension step may be for example 40, 45, 50, 55, 60, 65, 70, 75 or 80 min. According to one embodiment, the time of the final extension step is 55 to 65 min. Preferably, the time of the final extension step is about 60 min.

According to one embodiment, the temperature of the final extension step is in the range of 50 to 60 °C. The temperature of the final extension step may be for example 50, 51, 52, 53, 54, 55, 56, 57, 58, 59, or 60, °C. According to one embodiment, the temperature of the final extension step is 55 to 60 °C. Preferably, the temperature of the final extension step is about 60 °C.

According to one embodiment, the time of the annealing step is in the range of 80 to 100 s. The time of the annealing step may be for example 80, 81, 82, 83, 84, 85, 86, 87, 88, 89, 90, 91, 92, 93, 94, 96, 97, 98, 99, or 100 s. According to one embodiment, the time of the annealing step is 85 to 95 s. Preferably, the time of the annealing step is about 90 s.

According to one embodiment, the temperature of the annealing step is in the range of 50 to 70 °C. The temperature of the annealing step may be for example 50, 51, 52, 53, 54, 55, 56, 57, 58, 59, 60, 61, 62, 63, 64, 65, 66, 67, 68, 69, 70,°C. According to one embodiment, the temperature of the annealing step is 55 to 65 °C. Preferably, the temperature of the annealing step is about 60 °C.

There are different methods for separating the amplification products after the two of PCR. As shown in the examples, the standard procedure of separation by capillary sequencer is well-suited. This method is very cost-effective. Examples of capillary sequencers are CEQ8000 (Sciex), "SeqStudio Genetic Analyser" (Applied Biosystems) with 6 color channels or a sequencer of the company Promega "SpectrumCE" with 8 color channels. In the latter case, sample pooling allows the simultaneous analysis of 7 patient samples at the same cost.

With the selection of markers and particular primers for these markers as well as the buffer conditions, it is possible to amplify nine or more different markers in one PCR reaction. The number of X-linked SD markers may be 2 or 3. According to one embodiment, the method includes the amplification of at least three X-linked SD markers. Moreover, the number of STR markers identified in the method according to the invention may be 2, 3, 4, 5, 6, 7, 8, 9, 10 or more. According to one embodiment, the method includes the amplification of at least 3 STR markers, at least 4 STR markers, at least 5 STR markers or at least 6 STR markers. With all markers in one PCR reaction, the amplification products from a sample are separated in one color channel. Accordingly, the method according to the invention saves resources and costs making it very suitable for newborn screening.

The predefined range obtained from samples of karyotype 46,XX is determined by the arithmetic mean and the standard deviation of the peak height ratios or peak area ratios of the X-linked SD marker in healthy female subjects of karyotype 46,XX, with a P value of at least 95 %. In particular, the range is determined by a one-fold standard deviation, or a two-fold standard deviation or a three-fold standard deviation.

In this regard, the inventors have identified that the use of the Grubb's outlier test (Grubbs 1969) for determining the calculated peak height or peak area ratios fall into the predefined ranges achieves highly accurate results. Thus according to one embodiment, whether a calculated peak height or peak area ratio falls into the predefined ranges is determined by Grubb's outlier test. If a measured value is our of the predefined range obtained from samples of karyotype 46,XX, it is classified as Non 46,XX.

Due to genetic differences between individuals but also from one population to the next, it is advisable to determine a standard, i.e. the predefined range for every country or region in which the test should be offered. For determining the predefined ranges the number of healthy samples (46,XX) should be at least n = 15. The number of healthy sample may 15, 20, 25, 30, 35, 40 or more. According to one embodiment, the number of healthy samples at least 20. According to one embodiment, the number of healthy samples at least 25.

According to one embodiment, at least one X-linked SD marker is located an autosome and on the short arm of the X-chromosome (Xp). The Xp-linked marker may be SD-X3. According to one embodiment, at least one X-linked SD marker is located on the long arm of the X-chromosome (Xq). The Xq-linked marker may be SD-X16. The use of SD markers on the different arms of the X chromosome allows for example the detection of isochromosomes. According to one embodiment, the method comprises the amplification of at least Xp-linked marker and at least one Xq-linked marker. According to one embodiment, the method comprises the amplification of SD-X3 and SD-X16.

According to one embodiment, the method according includes the use of at least three X-linked SD markers. In this regard, at least one X-linked SD marker may be located on the X and the Y chromosome. Such marker is also referred to as XY linked marker. According to one embodiment, the XY-linked marker is SD-XY2. According to one embodiment, a peak height ratio or peak area ratio of the XY-linked SD marker outside of the predefined range obtained from samples of karyotype 46,XY is indicative of the diagnosis Turner-syndrome with mosaic 45,X0/46,XY or Klinefelter-syndrome 47,XXY and its mosaic variants.

According to one embodiment, the predefined range for SD-X3 is 0.88 to 1.26. According to a preferred embodiment, it is 0.85 to 1.26. According to one embodiment, the predefined range for SD-X16 is 0.88 to 1.13. According to a preferred embodiment, it is 0.86 to 1.15. According to one embodiment, the predefined range for SD-XY2 in the range of 1.01 to 1.26. According to a preferred embodiment, it is 0.99 to 1.27.

According to one embodiment, the amplification products of the SD markers and the STR markers have a length of less than 300 bp. The amplification products of the SD markers and the STR markers may have a length of 50, 60, 70, 80, 90, 100, 110, 120, 140, 150, 160, 180, 200, 220, 240, 260, 280, 300. According to one embodiment, the amplification products of the SD markers and the STR markers have a length of less than 250 bp. According to one embodiment, the amplification products of the SD markers and the STR markers have a length of less than 250 bp.

According to one embodiment, at least two STR markers are localized on Xp and at least two STR markers are localized on Xq. The use of STR markers on the different arms of the X chromosome, allows the detection of isochromosomes. Moreover, comparison of markers of the same arm increases the chance of detecting deletions in that arm of the chromosome.

According to one embodiment, STR markers are selected from GATA3108, DX101, DX6807, DX7132, DXS9902, and G09742. The positions of these markers in the X chromosome are identified in Fig. 5. According to one embodiment, two, three, four, five or all of these markers are used in the method. Preferably, all of the STR markers are used.

In the setup according to the invention, the STR markers are particular suitable for indicating or confirming a mosaic karyotype. Thus, according to one embodiment, the method further comprises a step f, determining the peak height ratio or peak area ratio for each heterozygous STR marker and comparing calculated ratios with a threshold obtained from heterozygous samples of karyotype 46,XX or 46,XY; wherein a peak height ratio or peak area ratio of the heterozygous STR marker above the threshold obtained from samples of karyotype 46,XX is indicative of a mosaic karyotype.

The threshold obtained from samples of karyotype 46,XX is determined by the arithmetic mean and the standard deviation of the peak height ratios or peak area ratios of the X-linked SD marker in healthy female subjects of karyotype 46,XX, with a P value of at least 95 %. In particular, the range is determined by a one-fold standard deviation, or a two-fold standard deviation or a three-fold standard deviation.

In this regard, the inventors have identified that the use of the Grubb's outlier test for determining the calculated peak height or peak area ratios fall into the predefined ranges achieves highly accurate results. Thus according to one embodiment, whether a calculated peak height or peak area ratio falls into the predefined ranges is determined by Grubb's outlier test. If a measured value is above the predefined range obtained from samples of karyotype 46,XX, it is mosaic.

Due to genetic differences between individuals but also from one population to the next, it is advisable to determine a standard, i.e. the predefined range for every region or country, in which the test should be offered. For determining the threshold the number of healthy samples (46,XX) should be at least n = 15. The number of healthy sample may 15, 20, 25, 30, 35, 40 or more. According to one embodiment, the number of healthy samples at least 20. According to one embodiment, the number of healthy samples at least 25.

According to one embodiment, the threshold for GATA31E08 is 1.30. According to one embodiment, the threshold for GATA31E08 is 1.32. According to one embodiment, the threshold for DXS101 is 1.57. According to one embodiment, the threshold for DXS101 is 1.62. According to one embodiment, the threshold for DXS6807 is 1.41. According to one embodiment, the threshold for DXS6807 is 1.44. According to one embodiment, the threshold for DXS7132 is 1.25. According to one embodiment, the threshold for DXS7132 is 1.28. According to one embodiment, the threshold for DXS9902 is 1.33. According to one embodiment, the threshold for DXS9902 is 1.37. According to one embodiment, the threshold for G09742 is 1.17. According to one embodiment, the threshold for G09742 is 1.20.

For the amplification of SD-X3 the primers with the nucleotide sequence SEQ ID NO: 1 and/or SEQ ID NO: 2 may be used. For the amplification of SD-X16 the primers with the nucleotide sequence SEQ ID NO: 3 and/or SEQ ID NO: 4 may be used. For the amplification of SD-XY2 the primers with the nucleotide sequence SEQ ID NO: 5 and/or SEQ ID NO: 6 may be used. For the amplification of GATA31E08 the primers with the nucleotide sequence SEQ ID NO: 7 and/or SEQ ID NO: 8 may be used. For the amplification of DXS101 SEQ ID NO: 9 and/or SEQ ID NO: 10 may be used. For the amplification of DXS6807 the primers with the nucleotide sequence SEQ ID NO: 11 and/or SEQ ID NO: 12 may be used. For the amplification of DXS7132 the primers with the nucleotide sequence SEQ ID NO: 13 and/or SEQ ID NO: 14 may be used. For the amplification of DXS9902 the primers with the nucleotide sequence SEQ ID NO: 15 and/or SEQ ID NO: 16 may be used. For the amplification of G09742 the primers with the nucleotide sequence SEQ ID NO: 17 and/or SEQ ID NO: 18 may be used.

### EXAMPLES

### Example 1 - Primer design

Primers are checked for SNPs in the primer binding region using, for example, "SNP-Check" (https://genetools.org/SNPCheck/snpcheck.htm), and the dimer formation and hairpin formation of primers can be checked using "Autodimer" (Vallone, et al. 2004) was tested. Adenylation of the amplicon, so-called A-tailing, was supported by synthetic additional sequences at the 5' end of the non-fluorescently labeled primer, for example 5'-GTTCTT:
The following primer sequences were used:

**Table 1. Marker and Primer sequences: Marker: designation of SD or STR markers, position: location on chromosomes in millions of base pairs [MB], primer sequence: DNA sequence of primers with f stands for forward primer and fl: Fluorescence-labeled primer, r = reverse primer, in bold are the additional nucleotides for A-tailing.**

| **Marker/Position [Mb]** | **Primer sequences (5' - 3')** |
|---|---|
| **SD_3X [77]** | f/fl: gtttctggttttgcctaggtccagtg (SEQ ID NO: 1); |
| | r: tgacaggtagttttgggtcag (SEQ ID NO: 2) |
| **SD_16X [44]** | f/fl: gtttctgttcttcagactgtagctccac (SEQ ID NO: 3); |
| | r: gtttctaggcactgtggttc (SEQ ID NO: 4) |
| **SD-XY2 [91]** | f/fl: aggcgagtagctgtctatga (SEQ ID NO: 5); |
| | r: **gttctt**cttttctaccactgaaacagagt (SEQ ID NO: 6) |
| **GATA31E08 [140]** | f/fl: gatagatgatagagatagatgatag (SEQ ID NO: 7); |
| | r: **gttctt**tgctcacttttatgtgtgtatgtatc (SEQ ID NO: 8) |
| **DXS101 [101]** | f/fl: cacattttactctaaatcagtccaaatatctc (SEQ ID NO: 9); |
| | r: **gttctt**ggcaaatcactccatggcacatgtat (SEQ ID NO: 10) |
| **DXS 6807 [47]** | f/fl: gagcaatgatctcatttgca (SEQ ID NO: 11); |
| | r: **gttct**taagtaaacatgtataggaaaaagct (SEQ ID NO: 12) |
| **DXS 7132 [64]** | f/fl: agcccattttcataataaatcc (SEQ ID NO: 13); |
| | r: **gttct**taatcagtgctttctgtactattgg (SEQ ID NO: 14), |
| **DXS 9902 [15]** | f/fl: tggagtctctgggtgaagag (SEQ ID NO: 15); |
| | r: **gttct**tcaggagtatgggatcaccag (SEQ ID NO: 16) |
| **G09742]92]** | f/fl: agtcatttcctctaacaagtctcc (SEQ ID NO: 17); |
| | r: **gttct**ttccagagagtcagaatcagtagg (SEQ ID NO: 18) |

### Example 2 - Direct PCR - Buffer selection

For direct PCR compositions that have high inhibitor tolerance are required. DNA amplification by direct PCR from dried blood stains (DBS) punches was tested.

The following buffer systems were used: HOT FIREPol^{®} MultiPlex Mix with 10 mM MgCl₂ (Solis Biodyne Estonia), S7Fusion (Biozym), Blutdirekt 2x PCR Mastermix (Bio&SELL), 5x Multiplex QPCR Mix (Bio&Sell), 5x Hemo KlenTaq Reaction buffer (NEB), Multiplex-PCR-Kit (Qiagen), Phusion High-Fidelity PCR Master Mix with HF Buffer (Thermofisher).

In the pipetting plan, the DBS punches were counted as DNA volume. A DBS punch with a diameter of 1.5 mm was counted as 1.5 µl DNA, a DBS punch with a diameter of 3 mm was counted as 3 µl DNA.

The temperature-time profile is adjusted as follows. Long annealing times as well as a long last extension step at 60 °C are chosen to achieve complete adenine addition to the PCR product and to obtain better data quality see table).

**Table 2: Temperature-time profile**

| **Step (35 Cycles)** | **Temperature** | **Time** |
|---|---|---|
| **Hot Start** | 95 °C | 15 min |
| **Denaturing** | 95 °C | 20 sec |
| **Annealing** | 60 °C | 90 sec |
| **Extension** | 72 °C | 20 sec |
| **Final Extension** | 60 °C | 60 min |

As shown in Fig. 1 using the HOT FIREPol^{®} MultiPlex Mix with 10 mM MgCl2 it was possible to obtain a high DNA yield for each of the marker products allowing a highly sensitive analyisis and detection of Non 46,XX or Non 46,XY karyotypes. With the other tested buffer systems a lower yield especially in the ranges > approx. 150 bp or no PCR product at all.

### Example 3 - Variation of Times and Temperatures of the Steps

In the following, the time and temperature profiles were varied:

**Table 3: Variation of the temperature-time profile**

| **Step (35 Cycles)** | **Temperature** | **Time** |
|---|---|---|
| **Hot Start** | 95 °C | 20 min |
| | 95 °C | 10 min |
| | 90 °C | 15 min |
| | | |
| **Denaturierung** | 95 °C | 20 sec |
| | 95 °C | 15 sec |
| | 90 °C | 20 sec |
| **Annealing** | 60 °C | 80 sec |
| | 60 °C | 70 sec |
| | 50 °C | 90 sec |
| | | |
| **Extension** | 72 °C | 18 sec |
| | 72 °C | 15 sec |
| | 70 °C | 20 sec |
| | | |
| **Final Extension** | 60 °C | 40 min |
| | 60 °C | 45 min |
| | 60 °C | 50 min |
| | 50 °C | 60 min |

Long annealing times of 90 sec (at a temperature of 60 °C) as well as a long last extension step at 60 °C (see protocol in table 1) brought the best PCR yield and data quality inter alia by achieving complete adenine addition to the PCR product. The variant protocols shown in Table 2 lead to a reduced yield and data quality.

### Example 4 - Sample-Pooling

The following color sample assignments and capillary sequencers were used.

**Table 4: Capillary sequencers and color sample assignments**

| **CEQ8000** | | **Seqstudio** | | **Spectrum CE** | |
|---|---|---|---|---|---|
| **Colour Label** | **Sample** | **Colour Label** | **Sample** | **Colour Label** | **Sample** |
| **Wellred D2** | Person 1 | 6-FAM | Person 1 | FL-6C | Person 1 |
| **Wellred D3** | Person 2 | TET | Person 2 | JOE-6C | Person 2 |
| **WellredD4** | Person 3 | VIC | Person 3 | TMR-6C | Person 3 |
| **Wellred D1** | Standard | HEX | Person 4 | CXR-6C | Person 4 |
| | | NED | Person 5 | TOM-6C | Person 5 |
| | | LIZ | Standard | WEN | Standard |

The gel electrophoresis run was started with default settings for STR fragment separation.

### Example 5 - Determination of SD Ratios for 46,XX and 46,XY

The SD ratio is the ratio of the peak height or peak area of the diagnostic marker relative to the reference marker. For optimal data analysis, the variation of this ratio should be as small as possible and known. For the quantification of X chromosomes, reference samples of healthy females (N >25) are analyzed and the mean value and standard deviation of the SD ratio are determined. From this it can be concluded for sample values whether these still lie in a tolerance range or are to be regarded as Non 46,XX or Non 46,XY.

The position of the SD makers SD-X16, SD-X3 and SD-XY2 on the X chromosome is shown in **Fig. 5****.**

**Table 5: SD Ratios for 46,XX and 46,XY**

| **Marker** | **SD-X16** | **SD-X3** | **SD-X16/SD-X3** | **SD-XY2** |
|---|---|---|---|---|
| **Number of subjects** | 25 | 25 | 25 | 3 |
| **Mean** | 1,07 | 1,00 | 1,07 | 1,13 |
| **Standard Deviation** | 0,07 | 0,05 | 0,09 | 0,04 |
| **Q95 (TAB)** | 2,66 | 2,66 | 2,66 | 2,66 |
| **Cut off [Q95) upper limit** | **1,26** | **1,126** | **1,32** | **1,255** |
| **Cut off [Q95) lower limit** | **0,88** | **0,88** | **0,82** | **1,013** |
| **Q99 (TAB)** | 3,01 | 3,01 | 3,01 | 3,01 |
| **Cut off [Q99] upper limit** | 1,29 | 1,15 | 1,37 | 1,27 |
| **Cut off [Q99] lower limit** | **0,85** | **0,86** | **0,78** | **0,99** |

A definition of the outlier limit calculated according to Grubb for this data set is shown in Fig. 3 a)-c).

### Example 6 - Determination of STR Ratios for mosaic state

In the heterozygous state of an X-STR marker, unequal allele peak heights refer to a mosaic state, for example 45,X0/46,XX or X-chromosomal aberrations. The analysis of heterozygous reference samples gives us a cut off value at which point we can speak of a Non 46,XX mosaic sample. The following table shows the calculation and cut off values for mosaics of each marker from the number of informative heterozygotes.

**Table 6: Allel-Ratios for Non-mosaic**

| **STR-Marker** | **GATA31 E08** | **DXS101** | **DXS6807** | **DXS7132** | **DXS9902** | **G09742** |
|---|---|---|---|---|---|---|
| **Number of subjects** | 24 | 13 | 16 | 21 | 21 | 19 |
| **Mean** | 1,13 | 1,17 | 1,19 | 1,11 | 1,10 | 1,09 |
| **Standard Deviation** | 0,06 | 0,17 | 0,08 | 0,05 | 0,09 | 0,03 |
| **Q95 (TAB)** | 2,64 | 2,33 | 2,44 | 2,58 | 2,58 | 2,53 |
| **Cut off [Q95)** | **1,30** | **1,57** | **1,41** | **1,25** | **1,33** | **1,17** |
| **Q99 (TAB)** | 2,99 | 2,61 | 2,75 | 2,91 | 2,91 | 2,85 |
| **Cut off [Q99]** | **1,32** | **1,62** | **1,44** | **1,28** | **1,37** | **1,20** |

A definition of the outlier limit calculated according to Grubb for each of the markers is shown in Fig. 4 a)-f).

### Example 7 - Newborn screening results

In the newborn screening, a DBS card is prepared according to the known protocol. A DBS punch of 3 mm in diameter is placed directly into a 30 µl PCR set-up and amplified with a SD-STR-QF multiplex PCR and separated on a capillary sequencer (see **Fig. 1****).** The multiplex primer for patient sample 1 can be labeled with a Well Red D4 dye, the multiplex primer for patient sample 2 can be labeled with a Well Red D3 dye, and multiplex primer for patient sample 3 can be labeled with a Wellred D2 dye. All three samples are pooled and separated in a capillary on a CEQ8000 (Sciex) or similar device. Each color channel corresponds to a patient sample, and each sample can be analyzed individually and independently.

The peak heights are determined by the analysis of the Genetic Analysis System of the sequencer, exported and read into Excel.

**Table 7: Results of the QF-PCR and following capillary electrophoresis**

| **Marker** | **Size of amplification product(s) [nt]** | **Peak-height [RFU]** |
|---|---|---|
| SD-X16 | 80;84 | 20124; 30886 |
| 31E08 | 104;108 | 80039; 46250 |
| G09742 | 139 | 78573 |
| SD-X3 | 154;158 | 68253; 85161 |
| DXS9902 | 188 | 33986 |
| SD-XY2 | X | 100597 |
| DXS101 | 236; 239 | 47399;66892 |
| DXS6807 | 268 | 59923 |
| DXS7132 | 290; 304 | 28720; 16326 |

Analysis for Non 46,XX or Non 46,XY i.e. gonosomal aneuploidies, aberrations and mosaics is performed using SD and STR ratios and reference values analyzed with the Grubb test (see **Fig. 2a****,** **2b** **Fig 3a****).**

Using the method according to the invention, the karyotype of seven subject was determined and two controls was determined.

**Table 8 - Karyotype detection of seven subjects**

| **ID** | **Heterozygous STR-Allele-Ratio** | **SD-X16 [44]** | **SD-X3 [77]** | **Xp/Xq** | **SD-XY2 [90]** | **Suspected of** |
|---|---|---|---|---|---|---|
| **female** | GATC31E08: 1.19; DXS101: 1.01; DXS6807: 1.21; DXS7132: 1.15; G09742: 1.18 | 1.14 | 0.97 | 1.00 | | |
| **male** | none detected | 0.67 | 0.53 | 1.26 | 1.04 | |
| **S1** | none detected | 0.61 | 0.52 | 1.17 | | Turner-Syndrom: SD-X16 and SD-X3 ratios less than reference threshold. |
| **S2** | GATC31E08: 2.47; DXS101: 1.62; DXS6807: 2.84; DXS7132: 3.22; G09742: 3.24 | 0.76 | 0.64 | 1.18 | | Turner-Syndrom: SD-X16 and SD-X3 ratios less than reference threshold 0.88. Mosaic: Allel-Ratio unbalanced higher than 1.30, 1.57,1.41, 1.251.17 respectively |
| **S3** | none detected | 0.62 | 0.54 | 1.14 | 0.34 | Turner-Syndrom: SD-X16 and SD-X3 ratios less than reference threshold 0.88. Mosaic 45,X0/46XY:SD-XY2-ratio less than 1.01 |
| **S4** | DXS7132: 1.09; GATC31E08: 1.04 | 0.65 | 0.94 | 0.69 | | Turner-Syndrom: 46,X,deIXp: SD-X16 ratio higher than reference threshold 0.99 and Xp/Xq ratio less than reference threshold 1.01or |
| **S5** | G09742: 1.07 | 0.65 | 0.87 | 0.747 | | Turner-Syndrom: 46,X,iXq: SD-X16 ratio higher than reference threshold 0.99 and Xp/Xq ratio less than reference threshold 1.01 |
| **S6** | GATC31E08: 2.01; DXS101: 1.97; DXS6807: 1.84; DXS7132: 2.01; DXS9902: 1,87 | 1.58 | 1.67 | 0.87 | | Triple X-Syndrom: SD-X16 and SD-X3 ratios higher than reference threshold 1.26 and 1.13, respectively. |
| **S7** | none detected | 1.13 | 0.98 | 1.15 | 0.62 | Klinefelter-Syndrom: SD-X16 and SD-X3 ratios in range of reference samples, but SD-XY2 higher less than reference male. |

The table shows X-SD marker ratios and X-STR allele ratios of the analysis of seven test samples and two controls. In column ID is written the sample name, followed by the allele ratios of heterzygous markers and the X-SD-ratios. Please note that the samples S4 and S5 cannot be differentiated between delXp or isochromosome Xq, both interpretations are possible. All samples are recognized as Non-46,XX or Non-46,XY, respectively.

To confirm the results, the karyotypes were determined via karyograms. Standard 30-cell karyotypes as recommended by the American College of Medical Genetics were performed to identify at least genotypes displaying 10% mosaicism with 95% confidence in all. Chromosomes were prepared from peripheral blood lymphocytes according to standard protocols. The table shows karyotypes of the samples and controls.

**Table 9: Results of karyotyping of samples and controls.**

| **ID** | **Karyo type (blood)** |
|---|---|
| **female** | 46,XX |
| **male** | 46,XY |

| | **Numeric aneuploidies with/without mosaic** |
|---|---|
| **S1** | 45,X0 |
| **S2** | mos 45,X [16]/47,XXX [1]/46,XX [13] |
| **S3** | mos 45,X [16]/46,X, dic(Y)[14] |

| | **Structural X chromosome aberrations (del/iso)** |
|---|---|
| **S4** | mos 46,X, i(X)(q10)[25]/45,X[5] |
| **S5** | mos 46,X,del(X)(p11.2)[26]/45,X[4] |

| | **Triple X** |
|---|---|
| **S6** | 47,XXX |

| | **Klinefelter** |
|---|---|
| **S7** | 47,XXY |

The results confirm accuracy of the method according to the invention.

Many modifications and other embodiments of the invention set forth herein will come to mind to the one skilled in the art to which the invention pertains having the benefit of the teachings presented in the foregoing description and the associated drawings. Therefore, it is to be understood that the invention is not to be limited to the specific embodiments disclosed and that modifications and other embodiments are intended to be included within the scope of the appended claims. Although specific terms are employed herein, they are used in a generic and descriptive sense only and not for purposes of limitation.

### EFERENCES

Borte, Stephan, Borte, Michael, Pöge, Andreas and Sack, Ulrich. "Nachweis von schweren angeborenen Immundefekten bei Neugeborenen: Diagnostisches Vorgehen bei Screening, Tracking und Follow-up" Laboratoriums Medizin, vol. 38, no. 3, 2014, pp. 107-120.
Czibere L, Burggraf S, Fleige T, et al. High-throughput genetic newborn screening for spinal muscular atrophy by rapid nucleic acid extraction from dried blood spots and 384-well qPCR. Eur J Hum Genet. 2020; 28(1):23-30).
AWMF Leitlinien: Neugeborenen-Screening auf angeborene Stoffwechselstörungen, Endokrinopathien, schwere kombinierte Immundefekte (SCID) und Mukoviszidose. 024-12, 2020).
Hassold, T., & Hunt, P. To err (meiotically) is human: The genesis of human aneu-ploidy. Nature Reviews Genetics 2, 280-291 (2001).
Gravholt CH, Andersen NH, Conway GS, Dekkers OM, Geffner ME, Klein KO, Lin AE, Mauras N, Quigley CA, Rubin K, Sandberg DE, Sas TCJ, Silberbach M, Söderström-Anttila V, Stochholm K, van Alfen-van derVelden JA, Woelfle J, Backeljauw PF. Clinical practice guidelines for the care of girls and women with Turner syndrome: pro-ceedings from the 2016 Cincinnati International Turner Syndrome Meeting. Eur J En-docrinol 2017; 177: G1-G70.)
Kathy Mann and Caroline Mackie Ogilvie (2012). QF-PCR: application, overview and review of the literature Prenatal Diagnosis 2012, 32, 309-314).
E. GRUBBS (1969). Procedures for Detecting Outlying Observations in Samples. Technometrics VOL. 11, No. 1 FEBRUARY 1969).
Vallone, P.M., and Butler, J.M. (2004) AutoDimer: a screening tool for primer-dimer and hairpin structures. Biotechniques 37(2): 226-231).

## Claims

1. A method for the *in vitro* detection of karyotypes with aberrations of the gonosomes or mosaics of said karyotypes in a human by quantitative fluorescence polymerase chain reaction (QF-PCR) using short tandem repeat (STR) markers and X-linked segmental duplication (SD) markers comprising the following steps:
a. providing a sample previously obtained from the human;
b. adding the sample to a PCR buffer system to and performing multiplex QF-PCR directly with the sample in a PCR buffer system using primer pairs for at least two X-linked SD markers and primer pairs for at least two STR markers located on the X-chromosome and to obtain amplification products;
c. separating the marker amplification products;
d. determining the peak heights or peak area of the separated amplification products;
e. calculating the peak height ratio or peak area ratio for each X-linked SD marker and comparing calculated ratios with a predefined range obtained from samples of karyotype 46,XX or 46,XY;
wherein a peak height ratio or peak area ratio of the X-linked SD marker outside of the predefined range obtained from samples of karyotype 46,XX is indicative of a karyotype with aberrations of the X chromosome or a mosaic of said karyotype.

2. The method according to claim 1, wherein the sample previously obtained from the human is selected from blood, saliva and urine preferably dry blood or oral mucosa swabs, more preferably the dry blood is in form of punches from dry blood cards.

3. The method according to claim 1 or 2, wherein the PCR buffer system contains bovine serum albumin (BSA), preferably in a concentration of 300 ng/ul to 600 ng/ul, more preferably the PCR buffer is 5X Hot FirePol Multiplex Mix with 10 mM MgCI2.

4. The method according to any one of the preceding claims, wherein PCR comprises a hot start step, a number of cycles of denaturing, annealing and extension and a final extension step, wherein:
• the time of the final extension step is in the range of 40 to 80 min, preferably 55 to 65 min, more preferably about 60 min;
• the temperature of the final extension step is in the range of 50 to 60 °C, preferably in the range of 55 to 60 °C, more preferably about 60 °C;
• the time of the annealing step is in the range of 80 to 100 s, preferably 85 to 95 s more preferably about 90 s;
• the temperature of the annealing step is in the range of 50 to 70 °C, preferably in the range of 55 to 65 °C, more preferably about 60 °C

5. The method according to any one of the preceding claims, wherein the amplification products are separated with a capillary sequencer and preferably amplification products from a sample are separated in one color channel.

6. The method according to claim 1, wherein the predefined range obtained from samples of karyotype 46,XX is determined by the arithmetic mean and the standard deviation of the peak height ratios or peak area ratios of the X-linked SD marker in healthy female subjects of karyotype 46,XX, with a P value of at least 95 %, and wherein the number of healthy samples is preferably at least n = 20.

7. The method according to any one of the preceding claims, wherein at least one X-linked SD marker is located an autosome and on the short arm of the X-chromosome (Xp), preferably the Xp-linked marker is SD-X3, and at least one X-linked SD marker is located on the long arm of the X-chromosome (Xq), preferably the Xq-linked marker is SD-X16.

8. The method according to any one of the preceding claims, wherein at least three X-linked SD markers are used and at least one X-linked SD marker is located on the X and the Y chromosome, preferably the XY-linked marker is SD-XY2, and wherein a peak height ratio or peak area ratio of the XY-linked SD marker outside of the predefined range obtained from samples of karyotype 46,XY is indicative of the diagnosis Turner-syndrome with mosaic 45,X0/46,XY; or Klinefelter-syndrome 47,XXY and its mosaic variants.

9. The method according to any one of the preceding claims, wherein the predefined ranges of the X-linked SD-markers are defined as follows:
• for SD-X3 in the range of 0,88 to 1,26, preferably in the range of 0.85 to 1.26
• for SD-X16 in the range of 0.88 to 1.13, preferably in the range of 0.86 to 1.15; and/or
• for SD-XY2 in the range of 1.01 to 1.26, preferably in the range of 0.99 to 1.27.

10. The method according to any one of the preceding claims, wherein the amplification products of the SD markers and STR markers have a length of less than 300 bp, preferably less than 250 bp, more preferably less than 200 bp.

11. The method according to any one of the preceding claims, wherein at least two STR markers are localized on Xp and at least two STR markers are localized on Xq.

12. The method according to any one of the preceding claims, wherein the STR markers are selected from GATA3108, DX101, DX6807, DX7132, DXS9902, and G09742, preferably all of these STR markers are used.

13. The method according to any one of the preceding claims, wherein the method further comprises the step
f. determining the peak height ratio or peak area ratio for each heterozygous STR marker and comparing calculated ratios with a threshold obtained from heterozygous samples of karyotype 46,XX or 46,XY;
wherein a peak height ratio or peak area ratio of the heterozygous STR marker above the threshold obtained from samples of karyotype 46,XX is indicative of a mosaic karyotype.

14. The method according to claim 13, wherein the threshold obtained from heterozygous samples of karyotype 46,XX or 46,XY predefined range obtained from samples of karyotype 46,XX is determined by the arithmetic mean and the standard deviation of the peak height ratios or peak area ratios of the STR marker in healthy female subjects of karyotype 46,XX, with a P value of at least 95 %, and wherein the number of healthy samples is preferably at least n = 10.

15. The method according to claim 14, wherein the thresholds are
• for GATA31E08 a value of 1.30, preferably 1.32;
• for DXS101 a value of 1.57, preferably 1.62;
• for DXS6807 a value of 1.41, preferably 1.44;
• for DXS7132 a value of 1.25, preferably 1.28;
• for DXS9902 a value of 1.33, preferably 1.37; and/or
• for G09742 a value of 1.17, preferably 1.20.

16. The method according to any one of the preceding claims, wherein the following primers are used:
• for SD-X3 SEQ ID NO: 1 and/or SEQ ID NO: 2;
• for SD-X16 SEQ ID NO: 3 and/or SEQ ID NO: 4;
• for SD-XY2 SEQ ID NO: 5 and/or SEQ ID NO: 6;
• for GATA31E08 SEQ ID NO: 7 and/or SEQ ID NO: 8;
• for DXS101 SEQ ID NO: 9 and/or SEQ ID NO: 10;
• for DXS6807 SEQ ID NO: 11 and/or SEQ ID NO: 12;
• for DXS7132 SEQ ID NO: 13 and/or SEQ ID NO: 14;
• for DXS9902 SEQ ID NO: 15 and/or SEQ ID NO: 16; and/or
• for G09742 SEQ ID NO: 17 and/or SEQ ID NO: 18.
